# FASCICULE DE BREVET EUROPEEN

(11) **EP 0 330 529 B1**
(45) Date de publication et mention de la délivrance du brevet: **31.03.1993**
(21) Numéro de dépôt: 89400197.3
(22) Date de dépôt: 25.01.1989
(51) Int. Cl.: C12P 41/00, C12P 7/40

(54) **Procédé de préparation d'acides aryl-2 propioniques optiquement actifs**
Verfahren zur Herstellung von optisch aktiven 2-Arylpropionsäuren
Process for the preparation of optically active 2-aryl-propionic acids

(30) Priorité: 27.01.1988 FR 8800923
(43) Date de publication de la demande: 30.08.1989
(73) Titulaire: RHONE-POULENC SANTE, 92160 Antony (FR)
(72) Inventeur: Cerbelaud, Edith, F-69005 Lyon (FR); Pétré, Dominique, F-69006 Lyon (FR)
(74) Mandataire: Pilard, Jacques

(56) Documents cités:
- EP-A- 0 227 078
- WO-A-86/07386
- FR-A- 1 342 844
- CHEMICAL ABSTRACTS, vol. 83, no. 3, 21 juillet 1975, page 471, résumé no. 27810q, Columbus, Ohio, US; S. RENDIC et al.: "Resolution of (+/-)-alpha-(3-benzoylphenyl-)propionic acid (Ketoprofen) and diastereometric interaction of its enantimers with some biological systems", & CHIMIA 1975, 29(4), 170-2
- CHEMICAL ABSTRACTS, vol. 81, no. 19, 11 novembre 1974, page 376, résumé no. 118551f, Columbus, Ohio, US; & JP-A-74 13 989 (DAIICHI SEIYAKU CO. LTD) 04-04-1974
- CHEMICAL ABSTRACTS, vol. 91, no. 11, 10 septembre 1979, page 610, résumé no. 895550d, Columbus, Ohio, US; & JP-A-79 46 887 (NITTO CHEMICAL INDUSTRY CO. LTD) 13-04-1979

## Description

La présente invention concerne un procédé de préparation d'acides aryl-2 propioniques optiquement actifs de formule générale : 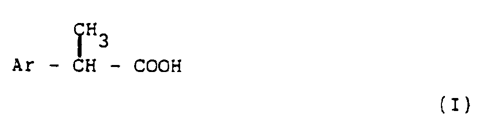
par hydrolyse énantiosélective des amides racémiques correspondants.

Dans la formule générale (I), Ar représente un radical aromatique monocyclique ou polycyclique éventuellement substitué ou un radical hétérocyclique aromatique éventuellement substitué.

Plus particulièrement, la présente invention concerne la préparation des énantiomères S des acides aryl-2 propioniques qui possèdent des propriétés anti-inflammatoires. Parmi les acides aryl-2 propioniques thérapeutiquement actifs peuvent être cités, par exemple, le kétoprofène, le naproxène, l'ibuprofène, le suprofène, le fénoprofène, le bénoxaprofène, le carprofène, le cicloprofène, le pirprofène, le flurbiprofène et le fluprofène.

Plus particulièrement encore, la présente invention concerne la préparation de l'énantiomère S (+) de l'acide (benzoyl-3 phényl)-2 propionique [kétoprofène S (+)].

Il est connu de préparer les énantiomères S des acides aryl-2 propioniques par oxydation stéréosélective d'aryl-2 propanes au moyen de microorganismes choisis parmi des champignons et des levures (EP-A-205215) ou par hydrolyse stéréospécifiques des esters racémiques au moyen d'une lipase d'origine extra-cellulaire ou microbienne (EP-A-227078).

Il est connu également (FR 7 901 803 / 2 447 359) de préparer des acides α-aminés optiquement actifs par hydrolyse biologique de nitriles α-aminés au moyen d'un agent contenant une nitrilase générale et une amidase L stéréospécifique ou d'amides α-aminés au moyen d'un agent contenant une amidase L stéréospécifique. Les agents utilisés sont des bactéries ou des préparations acellulaires d'origine bactérienne possédant une amidase L stéréospécifique qui proviennent de la mutation de souches possédant une amidase générale. L'hydrolyse des nitriles α-aminés ou des amides α-aminés conduit à un mélange de L α-aminoacide et de D α-aminoamide; le D α-aminoamide pouvant être hydrolysé en D α-aminoacide au moyen d'un agent possédant une amidase générale et ne possédant pas de racémase.

Il a maintenant été trouvé, et c'est ce qui fait l'objet de la présente invention, que les aryl-2 propionamides racémiques peuvent être hydrolysés énantiosélectivement en acides aryl-2 propioniques de formule générale (I) sous forme S au moyen de microorganismes ou d'enzymes sélectionnés en fonction de leur capacité à hydrolyser sélectivement l'α-phénylpropionamide racémique en acide α-phénylpropionique S.

La sélection des agents permettant l'hydrolyse énantiosélective des aryl-2 propionamides racémiques est effectuée en mettant en contact l'agent avec l'α-phénylpropionamide racémique dans un milieu convenable jusqu'à ce que 20% du produit soit converti puis en mesurant l'excès énantiomérique. Sont sélectionnés les agents qui hydrolysent, dans ces conditions, l'α-phénylpropionamide racémique en acide α-phénylpropionique S avec un excès énantiomérique supérieur à 65%.

Parmi les microorganismes qui conviennent particulièrement bien peuvent être citées des souches appartenant au genre Brevibacterium ou Corynebacterium et plus particulièrement Brevibacterium R 312 (CBS 717.73) et Corynebacterium N 771 (FERM P 4445) ou Corynebacterium N 774 (FERM 4446) qui permettent d'obtenir les acides aryl-2 propioniques S avec un excès énantiomérique en isomère S supérieur à 90%.

Il est surprenant de noter que, parmi ces microorganismes, Brevibacterium R 312, qui est décrit dans FR 7 901 803 / 2 447 359 et dans Advances in Biochemical Engineering, Vol. 14, p. 1-32 (1980) comme possédant une amidase générale non stéréospécifique hydrolyse stéréospécifiquement les aryl-2 propionamides racémiques alors que son mutant A₄ qui possède une amidase L stéréospécifique n'hydrolyse pas stéréosélectivement les aryl-2 propionamides racémiques.

Il y a lieu de noter que Corynebacterium N 771 et Corynebacterium N 774, qui sont décrits dans EP 0187680, hydrolysent par exemple le lactonitrile en acide D,L lactique et l'α-amino-phénylpropionitrile en D,L-phénylalanine sans observer de stéréosélectivité.

Selon la présente invention, le procédé est généralement mis en oeuvre en milieu aqueux ou hydroorganique homogène ou hétérogène, dans des conditions de température et de pH déterminées en fonction de la nature du microorganisme et de l'enzyme, en agitant une suspension de cellules ou d'extraits cellulaires du microorganisme et de l'aryl-2 propionamide racémique.

Le procédé conduit à un mélange de l'acide aryl-2 propionique S et de l'aryl-2 propionamide R, l'aryl-2 propionamide R pouvant être racémisé selon des techniques connues en aryl-2 propionamide racémique qui peut à nouveau être hydrolysé en acide aryl-2 propionique S dans les conditions données précédemment.

Par exemple, la racémisation de l'aryl-2 propionamide R peut être effectuée par chauffage avec de l'ammoniaque à une température comprise entre 80 et 160°C.

Lorsque le microorganisme utilisé possède la propriété d'hydrolyser les nitriles associée à la propriété d'hydrolyser énantiosélectivement l'α-phénylpropionamide, il est possible d'obtenir un acide aryl-2 propionique S soit à partir de l'aryl-2 propionitrile racémique soit à partir de l'aryl-2 propionamide racémique.

Le procédé selon la présente invention convient particulièrement bien pour préparer le kétoprofène S (+) à partir du (benzoyl-3 phényl)-2 propionamide racémique et éventuellement du (benzoyl-3 phényl)-2 propionitrile racémique.

Les exemples suivants, donnés à titre non limitatif, montrent comment l'invention peut être mise en pratique.

### EXEMPLE 1

a) La souche Brevibacterium R 312 (CBS 717.73) est cultivée en fiole agitée à 28°C pendant 14 heures dans un milieu dont la composition est la suivante :
- - Glucose: 10 g
- - (NH₄)₂SO₄: 5 g
- - KH₂PO₄: 1,01 g
- - Na₂HPO₄, 12H₂O: 1,64 g
- - K₂HPO₄: 0,82 g
- - Ca Cl₂, 2H₂O: 0,012 g
- - ZnCl₂: 0,0012 g
- - FeSO₄, 7H₂O: 0,0012 g
- - MnSO₄, H₂O: 0,0012 g
- - MgSO₄, 7H₂O: 0,5 g
- - Chlorhydrate de thiamine: 0,002 g
- - eau q.s.p.: 1000 cm3.

Cette préculture est utilisée pour ensemencer un milieu de culture ayant la même composition mais contenant en plus du N-méthylacétamide à la concentration de 20mM. La culture est effectuée en fiole agitée pendant 24 heures à 28°C. La biomasse obtenue est séparée par centrifugation puis elle est lavée deux fois par une solution de chlorure de sodium à 9 g/litre.

b) Un culot de centrifugation contenant 13 mg de cellules de Brevibacterium R 312, exprimés en matières sèches, est mis en suspension dans 2 cm3 de tampon phosphate (50 mM) à pH = 7. On ajoute 25 mg de phényl-2 propionitrile racémique (190 µmoles). Après 24 heures d'agitation à 25°C, le mélange réactionnel est dilué par additon de 23 cm3 d'un mélange acétonitrile-acide chlorhydrique N (90-10 en volumes). Les bactéries sont éliminées par centrifugation. La composition du surnageant est déterminée par chromatographie liquide à haute performance (CLHP).

Le surnageant contient :
119 µmoles de phényl-2 propionamide
62 µmoles d'acide phényl-2 propionique.

On ajoute du chlorure de sodium pour favoriser la séparation des phases aqueuses ou organiques. Après évaporation à sec de la phase organique, le résidu est repris par 20 cm3 d'un mélange chloroforme-soude 0,1N (1-1 en volumes).

La phase basique est acidifiée puis extraite au chloroforme.

La mesure du pouvoir rotatoire montre que l'excès énantiomérique en isomère S (+) est de 100%.

Après dérivation de l'acide phényl-2 propionique avec la R (+) α-méthylbenzylamine, l'analyse par CLHP montre que l'excès énantiomérique est de 96,4%.

### EXEMPLE 2

a) On cultive en fiole agitée à 28°C pendant 14 heures la souche Corynebacterium N 771 (FERM P 4445) dans un milieu ayant la composition suivante :
- - extrait de levure: 3 g
- - extrait de malt: 3 g
- - bactopeptone: 5 g
- - glucose: 10 g
- - FeSO₄, 7H₂0: 0,1 g
- - eau q.s.p.: 1 litre

et dont le pH est ajusté à 7,5 par addition de soude avant stérilisation.

Cette préculture est utilisée pour ensemencer au 1/40ème un milieu de même composition auquel on ajoute, à raison de 5 cm3 par litre, une solution de nitrile du kétoprofène dans l'acétonitrile (0,2 g/cm3).

Après incubation pendant 24 heures à 28°C en fiole agitée, la biomasse est séparée par centrifugation puis est lavée deux fois par une solution aqueuse de chlorure de sodium à 9 g/litre.

b) Un culot de centrifugation contenant 72 mg de cellules de Corynebacterium N 771, exprimés en matière sèche, est mis en suspension dans 2 cm3 de tampon phosphate (50 mM) à pH = 7. On ajoute 25 mg de phényl-2 propionamide racémique (167 µmoles) puis on agite pendant 24 heures à 25°C. Le mélange réactionnel est traité dans les conditions de l'exemple 1.

Le surnageant contient :
110 µmoles de phényl-2 propionamide
34 µmoles d'acide phényl-2 propionique.

Les excès énantiomériques de l'acide phényl-2 propionique mesurés par le pouvoir rotatoire et après dérivation avec la R (+) α-méthylbenzylamine sont respectivement de 100 et 95% en isomère S (+).

### EXEMPLE 3

Un culot de centrifugation, obtenu dans les conditions de l'exemple 1 a), contenant 13 mg de cellules de Brevibacterium R 312, exprimés en matière sèche, est mis en suspension dans 2 cm3 de tampon phosphate (50 mM) à pH = 7. On ajoute 25 mg de phényl-2 propionamide racémique (167 µmoles). On agite pendant 24 heures à 25°C. Le mélange réactionnel est traité dans les conditions de l'exemple 1.

Le surnageant contient :
95,2 µmoles de phényl-2 propionamide
56,1 µmoles d'acide phényl-2 propionique.

Les excès enantiomériques de l'acide phényl-2 propionique mesurés par le pouvoir rotatoire et après dérivation avec la R (+) α-méthylbenzylamine sont respectivement de 99 et 95% en isomère S (+).

### EXEMPLE 4

Un culot de centrifugation obtenu dans les conditions de l'exemple 1 a) contenant 66 mg de cellules de Brevibacterium R 312, exprimés en matières sèches, est mis en suspension dans 2 cm3 de tampon phosphate de potassium (50 mM) à pH = 7. On ajoute 25,9 mg de (benzoyl-3 phényl)-2 propionamide racémique (102 µmoles). On agite pendant 72 heures à 25°C. Le mélange réactionnel est dilué par addition de 23 cm3 d'un mélange acétonitrile - acide chlorhydrique N (90-10 en volumes). Les bactéries sont éliminées par centrifugation. Le surnageant, analysé par chromatographie liquide à haute performance, contient :
46 µmoles de kétoprofène
59 µmoles d'amide du kétoprofène.

On ajoute du chlorure de sodium pour favoriser la séparation des phases aqueuses et organiques. Après évaporation à sec de la phase organique, le résidu est repris par 20 cm3 d'un mélange chloroforme-soude 0,1N (1-1 en volumes).

La phase basique est acidifiée puis extraite au chloroforme. Après dérivation du kétoprofène avec la R (+) α-méthylbenzylamine, l'analyse par chromatographie liquide à haute performance du mélange des deux diastéréoisomères montre que l'excès énantiomérique du kétoprofène S (+) est de 93%.

### EXEMPLE 5

Un culot contenant 180 mg de cellules de Corynebacterium N 771 obtenu dans les conditions de l'exemple 2 a), exprimés en matière sèche, est mis en suspension dans 2 cm3 de tampon phosphate de potassium (50 mM) à pH = 7. On ajoute 25,3 mg d'amide du kétoprofène racémique (100 µmoles). On agite pendant 48 heures à 25°C puis on ajoute 23 cm3 d'un mélange acétonitrile - acide chlorhydrique N (90-10 en volumes). Les bactéries sont éliminées par centrifugation. Le surnageant, analysé par chromatographie liquide à haute performance, contient :
76,03 µmoles d'amide de kétoprofène
24,7 µmoles de kétoprofène.

On ajoute quelques mg de chlorure de sodium. Les phases aqueuse et organique se séparent. Après évaporation à sec de la phase organique, le résidu est repris par 20 cm3 d'un mélange chloroforme - soude 0,1 N (1-1 en volumes). La phase aqueuse est acidifiée puis extraite au chloroforme. Après dérivation du kétoprofène avec la R (+) α-méthylbenzylamine, l'analyse par chromatographie liquide à haute performance du mélange des deux diastéréoisomères montre que l'excès énantiomérique du kétoprofène S (+) est de 94%.

### EXEMPLE 6

Un culot de centrifugation, obtenu dans les conditions de l'exemple 1 a), contenant 24 mg de cellules de Brevibacterium R 312, exprimés en matière sèche, est mis en suspension dans 2 cm3 de tampon phosphate de potassium (50 mM) à pH = 7. On ajoute 23,5 mg de (benzoyl-3 phényl)-2 propionitrile racémique (nitrile du kétoprofène) soit 100 µmoles. On agite pendant 65 heures à 25°C puis on ajoute 23 cm3 d'un mélange acétonitrile - acide chlorhydrique N (90-10 en volumes). Les bactéries sont éliminées par centrifugation. Le surnageant, analysé par chromatographie liquide à haute performance, contient :
2,2 µmoles du nitrile du kétoprofène
53 µmoles d'amide du kétoprofène
44,7 µmoles de kétoprofène.

On ajoute quelques mg de chlorure de sodium. Les phases aqueuse et organique se séparent. Après évaporation à sec de la phase organique, le résidu est repris par 20 cm3 d'un mélange chloroforme - soude 0,1 N (1-1 en volumes). Après décantation, la phase organique est évaporée à sec. Le résidu (13,5 mg) contenant l'amide du kétoprofène est dilué dans 2 cm3 de chloroforme. La mesure du pouvoir rotatoire de cette solution montre que l'excès énantiomérique de l'amide du kétoprofène R (-) est de 84%.

La phase aqueuse, basique, est acidifiée puis extraite par du chloroforme. Après dérivation du kétoprofène avec la R (+) α-méthylbenzylamine, l'analyse par chromatographie liquide à haute performance du mélange des deux diastéréoisomères montre que l'excès énantiomérique du kétoprofène S (+) est de 96%.

### EXEMPLE 7

Un culot cellulaire de Corynebacterium N 774 (FERM P 4446) est préparé dans les conditions décrites dans l'exemple 2 a) pour la préparation d'un culot cellulaire de Corynebacterium N 771.

On met un culot cellulaire contenant 176 mg de cellules, exprimés en matière sèche, en suspension dans 2 cm3 de tampon phosphate de potassium (50 mM) à pH = 7. On ajoute 25,3 mg d'amide du kétoprofène racémique (100 µmoles). On agite pendant 48 heures à 25°C puis on traite le mélange réactionnel dans les conditions de l'exemple 1.

L'analyse du mélange réactionnel montre qu'il contient :
78,6 µmoles d'amide du kétoprofène
22,4 µmoles de kétoprofène.

L'excès énantiomérique du kétoprofène en isomère S (+) déterminé après dérivation est de 95%.

### EXEMPLE 8

Dans un autoclave de 5 cm3 contenant 1 cm3 d'ammoniaque à 28% (p/v) on introduit 20 mg d'amide du kétoprofène pur optiquement actif (79 µmoles). Après fermeture, l'autoclave est placé pendant 2 heures dans un four à 150°C. Après refroidissement le contenu de l'autoclave est versé dans 5 cm3 d'eau. On ajuste le pH à 1 par addition d'acide chlorhydrique 1 N. La phase aqueuse est extraite au chloroforme. La phase chloroformique est séchée sur sulfate de sodium. Après filtration, le volume est ajusté à 2 cm3 par concentration.

Le pouvoir rotatoire de cette solution chloroformique est nul.

L'analyse par CLHP montre que cette solution contient 72,2 µmoles d'amide du kétoprofène et 4,1 µmoles de kétoprofène.

## Revendications

1. Procédé de préparation des énantiomères S des acides aryl-2 propioniques de formule générale : 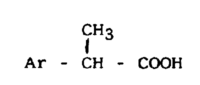 dans laquelle Ar représente un radical aromatique monocyclique ou polycyclique éventuellement substitué ou un radical hétérocyclique aromatique éventuellement substitué, caractérisé en ce que l'on hydrolyse énantiosélectivement les aryl-2 propionamides racémiques correspondants, éventuellement préparés in situ, en présence d'un microorganisme ou d'une enzyme issue de ce microorganisme sélectionné en fonction de sa capacité à hydrolyser sélectivement l'α-phénylpropionamide racémique en acide α-phénylpropionique S avec un excès énantiomérique supérieur à 65 %, puis sépare l'acide aryl-2 propionique S de l'aryl-2 propionamide R.

2. Procédé selon la revendication 1 caractérisé en ce que les microorganismes sont choisis parmi les Brevibacterium et les Corynebacterium.

3. Procédé selon la revendication 2 caractérisé en ce que les microorganismes sont choisis parmi Brevibacterium R 312 (CBS 717-73), Corynebacterium N 771 (FERM P 4445) et Corynebacterium N 774 (FERM P 4446).

4. Procédé selon la revendication 1 caractérisé en ce que le microorganisme possède une activité nitrilase associée à la capacité d'hydrolyser énantiosélectivement l'α-phénylpropionamide racémique en acide α-phénylpropionique S.

5. Procédé selon la revendication 4 caractérisé en ce que l'on hydrolyse directement l'aryl-2 propionitrile racémique en acide aryl-2 propionique S.

6. Procédé selon l'une des revendications 4 ou 5 caractérisé en ce que les microorganismes sont choisis parmi les Brevibacterium et les Corynebacterium.

7. Procédé selon la revendication 6 caractérisé en ce que les microorganismes sont choisis parmi Brevibacterium R 312 (CBS 771.73), Corynebacterium N 771 (FERM P 4445) et Corynebacterium N 774 (FERM P 4446).

8. Procédé selon l'une des revendications 1 à 7 pour la préparation de l'acide (benzoyl-3 phényl)-2 propionique S (+).

## Claims

1. Process for preparing the S enantiomers of the 2-arylpropionic acids of general formula: 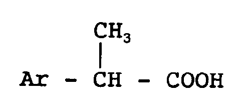 in which Ar represents an optionally substituted monocyclic or polycyclic aromatic radical or an optionally substituted aromatic heterocyclic radical, characterised in that the corresponding racemic 2-arylpropionamides, optionally prepared in situ, are hydrolysed enantioselectively in the presence of a microorganism, or of an enzyme originating from this microorganism, selected in accordance with its capacity to hydrolyse racemic α-phenylpropionamide selectively to(S)-α-phenylpropionic acid with an enantiomeric excess of more than 65%, and the (S)-2-arylpropionic acid is then separated from the (R)-2-arylpropionamide.

2. Process according to Claim 1, characterised in that the microorganisms are chosen from Brevibacterium species and Corynebacterium species.

3. Process according to Claim 2, characterised in that the microorganisms are chosen from Brevibacterium R 312 (CBS 717-73), Corynebacterium N 771 (FERM P 4445) and Corynebacterium N 774 (FERM P 4446).

4. Process according to Claim 1, characterised in that the microorganism possesses a nitrilase activity combined with the capacity to hydrolyse racemic α-phenylpropionamide enantioselectively to (S)-α-phenylpropionic acid.

5. Process according to Claim 4, characterised in that the racemic 2-arylpropionitrile is hydrolysed directly to the (S)-2-arylpropionic acid.

6. Process according to one of Claims 4 and 5, characterised in that the microorganisms are chosen from Brevibacterium species and Corynebacterium species.

7. Process according to Claim 6, characterised in that the microorganisms are chosen from Brevibacterium R 312 (CBS 771.73), Corynebacterium N 771 (FERM P 4445) and Corynebacterium N 774 (FERM P 4446).

8. Process according to one of Claims 1 to 7, for the preparation of (S)-(+)-2-(3-benzoylphenyl)propionic acid.

## Patentansprüche

1. Verfahren zur Herstellung von S-Enantiomeren der 2-Arylpropionsäuren der allgemeinen Formel 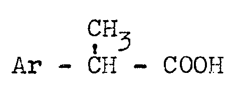 worin Ar einen gegebenenfalls substituierten, monocyclischen oder polycyclischen, aromatischen Rest oder einen gegebenenfalls substituierten, aromatischen, heterocyclischen Rest bedeutet, dadurch gekennzeichnet, daß man enantioselektiv die gegebenenfalls in situ hergestellten, entsprechenden racemischen 2-Arylpropionamide in Anwesenheit eines Mikroorganismus oder eines diesem Mikroorganismus entstammenden Enzyms, ausgewählt in Abhängigkeit von seiner Fähigkeit, selektiv racemisches α-Phenylpropionamid zu S-α-Phenylpropionsäure bei einem enantiomeren Überschuß von höher als 65% zu hydrolysieren, hydrolysiert, wonach man die S-2-Arylpropionsäure von dem R-2-Arylpropionamid abtrennt.

2. Verfahren gemäß Anspruch 1, dadurch gekennzeichnet, daß die Mikroorganismen unter Brevibacterium und Corynebacterium ausgewählt werden.

3. Verfahren gemäß Anspruch 2, dadurch gekennzeichnet, daß die Mikroorganismen unter Brevibacterium R 312 (CBS 717-73), Corynebacterium N 771 (FERM P 4445) und Corynebacterium N 774 (FERM P 4446) ausgewählt werden.

4. Verfahren gemäß Anspruch 1, dadurch gekennzeichnet, daß der Mikroorganismus eine Nitrilase-Aktivität besitzt, die an die Fähigkeit geknüpft ist, enantioselektiv racemisches α-Phenylpropionamid zu S-α-Phenylpropionsäure zu hydrolysieren.

5. Verfahren gemäß Anspruch 4, dadurch gekennzeichnet, daß man direkt racemisches 2-Arylpropionitril zu S-2-Arylpropionsäure hydrolysiert.

6. Verfahren gemäß einem der Ansprüche 4 oder 5, dadurch gekennzeichnet, daß die Mikroorganismen unter Brevibacterium und Corynebacterium ausgewählt werden.

7. Verfahren gemäß Anspruch 6, dadurch gekennzeichnet, daß die Mikroorganismen unter Brevibacterium R 312 (CBS 771.73), Corynebacterium N 771 (FERM P 4445) und Corynebacterium N 774 (FERM P 4446) ausgewählt werden.

8. Verfahren gemäß einem der Ansprüche 1 bis 7, zur Herstellung von S(+)-2-(3-Benzoylphenyl)-propionsäure.
